Europäisches Patentamt

European Patent Office

Office européen des brevets

(19)

(11) Numéro de publication : **0 345 315 B1**

(12) **FASCICULE DE BREVET EUROPEEN**

(45) Date de publication du fascicule du brevet :
24.03.93 Bulletin 93/12

(51) Int. Cl.⁵ : **C07K 7/10,** C12P 21/00,
**A61K 39/395, G01N 33/574**

(21) Numéro de dépôt : **88910051.7**

(22) Date de dépôt : **28.10.88**

(86) Numéro de dépôt international :
**PCT/FR88/00531**

(87) Numéro de publication internationale :
**WO 89/03845 05.05.89 Gazette 89/10**

(54) **PEPTIDES DERIVES DE LA PROTEINE PS2.**

(30) Priorité : **30.10.87 FR 8715060**

(43) Date de publication de la demande :
**13.12.89 Bulletin 89/50**

(45) Mention de la délivrance du brevet :
**24.03.93 Bulletin 93/12**

(84) Etats contractants désignés :
**AT BE CH DE FR GB IT LI LU NL SE**

(56) Documents cités :
**EP-A- 0 118 365**
**Nucleic Acids Research, vol. 15, no. 4, 25 février 1987, IRL Press, Oxford-Washington DC, J.J. Jeltsch et al.: "Structure of the human oestrogen-responsive gene pS2", pages 1401-1414, voir page 1402, lignes 9-21**
**Science, vol. 241, 5aout 1988, M.C. Rio et al.: "Breast cancer - associateed pS2 protein: synthesis and secretion by normal stomach mucosa", pages 705-708, voir le document en entier**

(56) Documents cités :
**Annals of the New York Academy of Sciences, vol. 464, 1986, The New York Academy of Sciences (New York, US), B. Gairard et al.: "Estrogen-inducible gene in human breast cancer", pages 443-447, see page 444, results and conclusion**
**Endocinology vol. 121 (1987), 1759 ff.**

(73) Titulaire : **ADEREGEM**
**23, rue Montpensier**
**F-75001 Paris (FR)**

(72) Inventeur : **CHAMBON, Pierre**
**200, rue du Docteur-A.-Schweitzer**
**F-67113 Blaesheim (FR)**
Inventeur : **RIO, Marie-Christine**
**5, rue de l'Argile**
**F-67400 Illkirch-Graffenstaden (FR)**
Inventeur : **BELLOCQ, Jean-Pierre**
**39, rue de l'Engelbreit**
**F-67200 Strasbourg (FR)**

(74) Mandataire : **Ores, Irène et al**
**CABINET ORES 6, Avenue de Messine**
**F-75008 Paris (FR)**

EP 0 345 315 B1

## Description

La présente invention est relative à l'identification de la séquence de la protéine pS2 sécrétée dans différents tissus et fluides humoraux humains, ainsi qu'a des fragments peptidiques de cette protéine ; la présente invention est également relative à des anticorps polyclonaux et monoclonaux anti-pS2 et anti - fragments peptidiques de la protéine pS2, et aux applications de cette protéine, peptides et anticorps pour le diagnostic et la détection de divers états pathologiques et notamment de cancers du sein hormono-dépendants, de cancers ou d'ulcères de l'estomac.

L'hormono-dépendance du cancer du sein est un fait connu depuis 1896 lorsque Beatson [Lancet (1986), 2, 104-107] rapportait deux observations de régression de tumeurs inopérables après ovariectomie chez des femmes encore réglées. Il est maintenant bien établi qu'environ un tiers des cancers mammaires répondent à des hormones et régressent à la suite de manipulations hormonales variées.

Jusqu'à ces dernières années il n'existait pas de test biochimique pour identifier les femmes atteintes de cancer hormono-dépendant et qui pouvaient donc bénéficier d'une thérapeutique endocrinienne. En 1971, Jensen et coll. [NATL CANCER INSTI. MONOGR. (1971) 34. 55-70] ont, les premiers. montre que la mesure des récepteurs cestrogéniques dans un prélèvement tumoral pouvait être utile pour prédire la réponse à une surrénalectomie. Cette observation a depuis lors été amplement confirmée puisque 50 à 65% des femmes dont les tumeurs contiennent des récepteurs oestrogéniques (RE) répondent à une thérapeutique endocrinienne, alors que celles dont les tumeurs en sont dépourvues n'ont, au mieux, que 10% de chances d'être améliorées par le traitement hormonal.

En effet, si les cellules cancéreuses possèdent des sites ayant une forte affinité pour une hormone (c'est-à--dire un récepteur), sites normalement présents dans la glande mammaire, leur croissance, comme celle des cellules normales, peut être régulée par l'environnement hormonal. Au contraire, si les cellules cancéreuses perdent leurs récepteurs au cours de la transformation maligne, elles ne sont plus reconnues comme cellules cibles. Cependant le résultat du dosage des récepteurs oestrogéniques (RE) ne permet pas de prédire parfaitement la réponse à une hormonothérapie puisque 55% à 65% seulement des femmes dont les tumeurs possèdent des récepteurs répondent favorablement à un traitement hormonal. Une des explications réside dans le fait , qu'au cours de la dédifférentiation, certaines tumeurs peuvent perdre leurs récepteurs oestrogéniques. Si ceux-ci persistent, elles peuvent conserver la capacité de lier l'estradiol mais sont incapables de poursuivre les étapes ultérieures de l'action oestrogénique. Il s'agit dans les deux cas de tumeurs qui sont hormonalement autonomes ou hormono-résistantes. Pour vérifier cette dernière hypothèse on est allé au-delà de l'étape initiale, c'est-à-dire de la liaison au récepteur cytosolique et on a recherché les produits terminaux de l'action intracellulaire des oestrogènes. C'est le cas, par exemple, du récepteur de la progestérone (RP) puisque sa synthèse est sous la dépendance des oestrogènes dans les cellules MCF-7 (lignée cellulaire dérivée d'un cancer du sein humain), et probablement dans les cellules cancereuses mammaires humaines in vivo. Effectivement, si l'on tient compte du taux des récepteurs progestéroniques, les taux de rémission des cancers sous traitement hormonal sont de l'ordre de 65%. De nombreux essais cliniques ont établi que 80% des femmes dont les tumeurs possédaient les deux récepteurs répondent à une hormonothérapie. Par contre si la tumeur contient des récepteurs oestrogéniques mais pas de récepteurs progestéroniques, la probabilité de réponse ne dépasse pas un tiers des cas. Les récepteurs progestéroniques (RP) ajoutent donc une information supplémentaire sur la prédiction de la réponse thérapeutique.

Les oestrogènes stimulent la synthèse d'un grand nombre de protéines qui sont libérées dans le milieu d'incubation de lignées cellulaires telles que les MCF-7. La plupart des protéines sécrétées sont détectées dans le milieu d'incubation avec ou sans oestradiol mais leur activité est très nettement augmentée en présence de cette hormone. Elles correspondent à des poids moléculaires de 37 000, 46 000, 54 000, 60 000 M [MAIRESSE et al. in RECENT RESULTS IN CANCER RESEARCH, G. LECLERCQ, S. TOMA, R. PARIDAENS, J.C. HEUSEN, Vol. 91, (1984) 301-306]. Certaines d'entre elles (46 000, 54 000 et 60 000 M) sont identiques aux protéines cytosoliques. Une protéine de 50 000 M est plus abondante dans le milieu d'incubation traité par oestradiol maie les travaux de MAIRESSE démontrent qu'il s'agit d'une stimulation de la sécrétion de cette protéine sous l'action d'une hormone, plutôt qu'une induction. Par ailleurs, ces protéines stimulées existent également dans le milieu d'incubation des cellules MCF-7 et sont présentes également dans le milieu d'incubation des cellules Evsa-T oestrogéno-indépendantes, mais l'oestradiol dans ce dernier milieu n'a aucun effet sur les synthèses et(ou leur sécrétion. Il ne s'agit donc pas de l'induction d'un nouveau produit sous influence hormonale mais seulement de l'augmentation de la concentration d'un produit existant.

ROCHEFORT (même publication que ci-dessus p. 289-294) a mis en évidence un taux élevé de protéine 52 K dans le milieu d'incubation et a montré que l'induction de cette protéine était spécifique de l'action 'de l'oestradiol à des concentrations physiologiques alors que la progestérone et la dexaméthasone étaient inactives. Le tamoxifène qui inhibe la croissance cellulaire n'induit pas la sécrétion de la protéine 52 K et prévient l'action de l'oestradiol dans un rapport molaire de 10. L'un de ses métabolites, le mono-hydroxy-tamoxifène est 200 fois plus actif que le tamoxifène pour bloquer la croissance cellulaire et la sécrétion de 52 K dans les cellules MCF-7. Plus récemment, cette même équipe a pu constater que dans une variante des cellules MCF-7, les cellules R-27 qui possèdent des RE et RP mais dont la croissance échappe à l'action des anti-oestrogènes, la protéine 52 K continue à être sécrétée en présence de tamoxifène ou de mono-hydroxytamoxifène.

Une équipe de Chercheurs comprenant certains des Inventeurs de la présente Demande a visé à l'expression de gènes spécifiques. A partir d'une banque de ADNc construite à partir de cellules MCF-7 induites par l'oestradiol, il a été possible de procéder à un clonage différentiel des ADNc correspondant au ARNm synthétisé en présence de cette hormone. A l'aide d'une sonde de ADNc fabriquée à partir de cellules poussant en présence et en l'absence d'hormone, un clone ADNc correspondant à un ARNm qui n'est présent que dans les cellules MCF-7 cultivées en présence d'oestradiol, a pu être isolé. Il a été dénommé pS2. Les Auteurs ont déduit de la détermination de la séquence nucléotidique du ADNc cloné qu'il s'agit d'une protéine comprenant 84 acides aminés, de faible poids moléculaire, 9140 Daltons, [JAKOWLEW et al. NUCLEIC ACIDS RES, (1984) 12, 2861-2878]. La régulation hormonale qui s'exerce sur le gène pS2 est située au niveau transcriptionnel. Le gène n'est pas transcrit en l'absence d'oestradiol, alors que l'accumulation de ARNm est nette huit heures après l'addition de l'hormone au milieu de culture. Les Auteurs n'avaient cependant pas encore isolé la protéine pS2. Le criblage de la banque de ADNc construite à partir de cellules MCF-7, induit par l'oestradiol, a permis d'isoler également deux autres clones 36 B4 et 3 A5. Aucune régulation hormonale ne s'exerce au niveau transcriptionnel de leur gène correspondant. Ces deux clones ont été appelés clones "constants". Les sondes 36 B4 et 3 A5 peuvent donc être utilisées pour apprécier la quantité de ARNm totaux présents, alors que la sonde pS2 correspond à un ARN spécifique, oestrogeno-induit, des cellules MCF-7. le pS2 ARN n'est pas présent dans le ARN extrait des cellules de cancer du sein humain T 47 D, qui contiennent à la fois les récepteurs oestrogéniques et progestéroniques, mais dans lesquelles la présence de ce dernier récepteur est constitutive. A l'inverse, l'ARN 36 B4 est présent dans les cellules T 47 D.

JELTSCH et al. [NUCLEIC ACID RES, (1987), 15, p. 1401-1414] ont ultérieurement cloné le gène pS2 humain à partir d'ADN de cellules de placenta et de cellules de la lignée MCF-7, ont étudié sa structure et ont établi et donc vérifié la séquence en nucléotides du gène pS2 à partir du clone pS2M obtenu à partir de ladite lignée cellulaire, et à partir du clone pS2P obtenu à partir de cellules placentaires.

L'observation que l'ARN pS2 est exprimé dans la liguée cellulaire MCF-7 dérivée d'un cancer du sein humain, mais ne l'est pas dans la lignée cellulaire T 17D, indique une voie d'identification de cancers du sein hormono-dépendants. C'est la raison pour laquelle les Inventeurs ont cherché à vérifier si l'expression du gène pS2 peut constituer un marqueur supplémentaire pour le dépistage des cancers du sein hormono-dépendants.

Plus récemment [RIO et al., SCIENCE (1988) 241, p. 705-707] la protéine pS2 a été mise en évidence dans les cellules à mucus de l'épithélium gastrique. La protéine sécrétée dans le liquide gastrique présente une migration électrophorétique identique à celle observée pour la protéine sécrétée par les cellules MCF-7 et les travaux rapportés dans l'article précité montrent que la taille et la séquence des ARNm isolés des deux tissus sont strictement identiques.

Il y a lieu de rappeler que la séquence (I) déterminée pour la protéine pS2 à partir de l'ARNm, comprend 84 acides aminés, a un poids moléculaire de l'ordre de 9140 Daltons, et est la suivante :

```
1                                                                10
MET ALA THR MET GLU ASN LYS VAL ILE CYS ALA LEU VAL LEU

                              20
VAL SER MET LEU ALA LEU GLY THR LEU ALA GLU ALA GLN THR
    30                                                   40
GLU THR CYS THR VAL ALA PRO ARG GLU ARG GLN ASN CYS GLY
                                   50
PHE PRO GLY VAL THR PRO SER GLN CYS ALA ASN LYS GLY CYS
           60                                           70
CYS PHE ASP ASP THR VAL ARG GLY VAL PRO TRP CYS PHE TYR
                                   80
PRO ASN THR ILE ASP VAL PRO PRO GLU GLU GLU CYS GLU PHE

                                             (I)
```

Or, les Inventeurs ont pu établir qu'il ne s'agit pas de la forme sécrétée du peptide pS2.

La présente invention s'est en conséquence donné pour but d'identifier la séquence primaire complète effective du peptide pS2 sécrétée dans différents tissus, dans le but de permettre sa production par genie génétique, notamment par synthèse dans un vecteur et un hôte appropriés, et d'élaborer des anticorps utilisables, entre autres, pour le dépistage et le diagnostic d'états pathologiques.

La présente invention a pour objet un peptide caractérisé en ce qu'il est constitué par un fragment correspondant à 60 acides aminés de la protéine de séquence (I) précédemment identifiée, en ce que ce peptide correspond à la forme sécrétée et en ce qu'il commence par la séquence GLU - ALA - GLN, GLU étant situé en vingt cinquième position après la MET N-terminale de la protéine de séquence (I) initialement identifiée, et en ce que son poids moléculaire est d'environ 6 600 Da.

Conformément à la présente invention le peptide pS2 de 60 acides aminés comprend la séquence (D) suivante :

```
                        30
GLU ALA GLN THR GLU THR CYS THR VAL ALA PRO ARG GLU ARG
    40                                               50
GLN ASN CYS GLY PHE PRO GLY VAL THR PRO SER GLN CYS ALA
                                   60
ASN LYS GLY CYS CYS PHE ASP ASP THR VAL ARG GLY VAL PRO
           70                                           80
TRP CYS PHE TYR PRO ASN THR ILE ASP VAL PRO PRO GLU GLU
GLU CYS GLU PHE

                                     (D)
```

Egalement conformément à la présente invention, le peptide de séquence (D) comprend quatre sites de coupure par la trypsine, qui sont situés après les acides aminés basiques $Arg_{36}$, $Arg_{38}$, $Lys_{54}$ et $Arg_{63}$.

La présente invention a également pour objet des peptides dits "peptides trypsiques" obtenus par coupure du peptide de séquence (D) aux sites de coupure par la trypsine.

Au nombre de ces peptides trypsiques, l'invention englobe notamment :

- un peptide trypsique correspondant aux 12 acides aminés N-terminaux du peptide de séquence (D)

- un peptide trypsique correspondant à la séquence d'acides aminés allant de la GLN$_{39}$ à la LYS$_{54}$
- un peptide trypsique correspondant à la séquence d'acides aminés allant de la GLY$_{55}$ à l'ARG$_{63}$.
- un peptide trypsique correspondant aux 21 acides aminés C-terminaux dudit peptide.

La présente invention a également pour objet un peptide caractérisé en ce qu'il constitue le peptide signal lors de la sécrétion de la protéine pS2, notamment dans le cas de cancers du sein et d'états pathologiques de l'estomac, et comprenant les 24 amino-acides N-terminaux de la protéine pS2.

La présente invention a en outre pour objet un peptide caractérisé en ce qu'il est constitué par un fragment correspondant aux 31 acides aminés C-terminaux du peptide de séquence (D) et en ce que son poids moléculaire est d'environ 3450 Da.

La présente invention a en outre pour objet un peptide caractérisé en ce qu'il est constitué par un fragment correspondant aux 30 acides aminés N-terminaux du peptide de séquence (D) et en ce que son poids moléculaire est d'environ 3300 Da.

La présente invention a en outre pour objet un peptide caractérisé en ce qu'il est constitué par un fragment correspondant aux 28 acides aminés de l'extrémité C-terminale du peptide de séquence (D) et en ce que son poids moléculaire est d'environ 3100 Da.

Conformément à la présente invention le peptide signal présente la séquence (G) en acides aminés la suivante :

```
1                                          10
MET ALA THR MET GLU ASN LYS VAL ILE CYS ALA LEU VAL LEU
                    20
VAL SER MET LEU ALA LEU GLY THR LEU ALA
```

( G )

Conformément à la présente invention les fragments peptidiques dits "peptides trypsiques" correspondent aux séquences en acides aminés (H), (J), (K) et (L) suivantes :

30
GLU ALA GLN THR GLU THR CYS THR VAL ALA PRO ARG

(H)

40                                    50
GLN ASN CYS GLY PHE PRO GLY VAL THR PRO SER GLN CYS ALA ASN LYS.

(J)

60
GLY CYS CYS PHE ASP ASP THR VAL ARG

(K)

70
GLY VAL PRO TRP CYS PHE TYR PRO ASN THR ILE ASP VAL PRO

80
PRO GLU GLU GLU CYS GLU PHE

(L)

Conformément à l'invention la composition (C) du peptide qui comprend 31 acides aminés, est la suivante :

60
LYS GLY CYS CYS PHE ASP ASP THR VAL ARG GLY VAL PRO TRP CYS ...
70                                    80
PHE TYR PRO ASN THR ILE ASP VAL PRO PRO GLU GLU GLU CYS GLU

PHE.

(C)

Egalement conformément à l'invention la composition (E) du peptide qui comprend 30 acides aminés est la suivante :

30
GLU ALA GLN THR GLU THR CYS THR VAL ALA PRO ARG GLU ARG
40                                    50
GLN ASN CYS GLY PHE PRO GLY VAL THR PRO SER GLN CYS ALA

ASN LYS

(E)

Egalement conformément à l'invention la composition (F) du peptide qui comprend 28 acides aminés est la suivante :

```
             60                                                    70
CYS PHE ASP ASP THR VAL ARG GLY VAL PRO TRP CYS PHE TYR
                                          80
PRO ASN THR ILE ASP VAL PRO PRO GLU GLU GLU CYS GLU PHE
```

(F)

La présente invention a également pour objet des anticorps polyclonaux anti-pS2 caractérisés en ce qu'ils sont obtenus par immunisation de lapins ou autres animaux appropriés, contre la protéine pS2 et ses fragments.

La présente invention a en outre pour objet des anticorps monoclonaux anti-pS2 caractérisés en ce qu'ils sont obtenus par clonage d'hybridomes résultant de la fusion de cellules de rates de mammifères appropriés immunisés par des injections convenables de la protéine pS2 ou de fragments de celle-ci, avec des cellules d'un myélome approprié.

Bien entendu, tous autres procédés d'obtention d'anticorps monoclonaux, entrent dans le cadre de la présente invention, dans la mesure où ils permettent d'obtenir les anticorps monoclonaux anti-pS2 qui sont protégés dans le cadre de la présente invention.

La présente invention a également pour objet un procédé d'obtention du peptide de formule (D) purifié à partir d'un liquide d'origine biologique , caractérisé en ce qu'on précipite les protéines contenues dans ce liquide par de l'acétone à basse température, notamment à -20°C environ , on purifie le culot renfermant le peptide, par chromatographie pour récupérer les fractions contenant le peptide, qui sont éventuellement congelées et/ou concentrées.

Selon un mode de mise en oeuvre du procédé d'obtention du peptide de formule (D) conforme à la présente invention, préalablement à la purification du liquide d'origine biologique, on part de cellules MCF-7 qui sont mises en culture en présence d'oestradiol, le surnageant de culture est recueilli et débarrassé des débris essentiels qu'il contient, puis soumis au processus de purification susdit.

Selon un autre mode de mise en oeuvre du procédé d'obtention du peptide de formule (D) conforme à l'invention, le liquide biologique mis en oeuvre est du suc gastrique contenant ledit peptide sécrété par les tissus gastriques que l'on neutralise par un tampon approprié à pH 9,6 environ, avant de le soumettre au processus de purification susdit.

Selon un mode de mise en oeuvre avantageux du procédé d'obtention du peptide de formule (D), ce dernier est isolé par immuno-purification à partir de liquides biologiques ou d'extraits de cellules contenant ledit peptide, à l'aide d'anticorps dirigés contre la protéine pS2 ou contre des fragments de celle-ci.

La présente invention a, de plus, pour objet un procédé de préparation de la protéine pS2 et de fragments de celle-ci, par voie de synthèse, notamment par synthése chimique ou par biosynthèse.

On peut utiliser, en particulier, la méthode en phase solide mise au point par MERRIFIELD; (La méthode de MERRIFIELD est décrite dans "Special Methods in peptide synthesis" publié en 1980 par E. GROSS et J. MEIENHOPER dans "THE PEPTIDES : Analysis, synthesis, biology", vol. 2, Partie A. p. 1-254, Academic Press, New-York), ou toute autre méthode de synthèse (Biosynthèse, par génie génétique, notamment).

La présente invention a également pour objet une méthode de dépistage et de diagnostic de l'état pathologique d'un organe qui provoque l'expression par ledit organe du peptide de formule (D) ou de ses précurseurs ou de ses fragments, caractérisée en ce que la présence d'un de ces peptides est déterminée par méthode immunologique dans des prélèvements biologiques, à l'aide d'anticorps dirigés contre l'un desdits peptides.

Selon un autre mode de mise en oeuvre de l'invention, on effectue le dépistage et le diagnostic sur des fluides corporels.

Selon un mode de réalisation avantageux de la méthode de dépistage et 'de diagnostic conforme à l'invention, l'expression du gène pS2 est déterminée par immunocytochimie.

Selon un autre mode de réalisation avantageux de ladite méthode de détection, l'expression du gène pS2 est déterminée dans des prélèvements de tumeurs.

Selon encore un autre mode de réalisation avantageux de la méthode de détection et de diagnostic conforme à l'invention, les prélèvements de. tumeurs mis en oeuvre peuvent être, entre autres, des coupes histologiques ou des cytosols.

Selon une disposition avantageuse de ce mode de réalisation, les prélèvements de tumeurs mis en oeuvre sont des coupes histologiques conservées et stabilisées par enrobage dans de la paraffine et fixées à la formaline, qui confèrent à la méthode de diagnostic une sensibilité très grande, un faible bruit de fond et préservent la structure histopathologique.

Selon un mode de réalisation avantageux de la méthode de détection et de diagnostic conforme à l'invention, donné à titre d'exemple, la méthode immunocytochimique est mise en oeuvre par incubation desdits prélèvements avec des anticorps de lapin, ou autre animal approprié, poly- ou monoclonaux anti-pS2 ou anti-fragments de pS2, précédée d'une incubation avec du sérum de mouton (ou d'un autre animal approprié) pour réduire les colorations non-spécifiques, et suivie d'une pluralité d'incubations successivement avec des IgG de mouton anti-lapin, puis avec un système de marquage et de révélation approprié de l'anticorps.

Selon une disposition avantageuse de l'invention ledit système de marquage et de révélation de l'anticorps peut comprendre un système PAP (peroxydase-antiperoxydase) de lapin, avec lequel lesdits prélèvements sont alors incubés, après quoi ils sont incubés avec du DAB chlorhydrate de 3,3'-diamino-benzidine), ces opérations étant éventuellement suivies d'une coloration de la coupe par de l'hématoxyline ou analogue, la coloration obtenue étant estimée sur une échelle d'intensité de coloration et la proportion de cellules tumorales colorées déterminée en pourcentage des cellules tumorales totales (1-100%), tandis que l'indice de coloration est obtenu en multipliant le pourcentage de cellules colorées par le score de coloration (1-300).

L'invention a également pour objet un kit prêt à l'emploi pour le dépistage et le diagnostic de l'état pathologique d'un organe, par dosage immunologique, caractérisé en ce qu'il comprend :
- des doses appropriées d'anticorps polyclonaux ou monoclonaux dirigés contre la protéine pS2 et/ou ses fragments ;
- des doses appropriées de protéine pS2 et/ou de ceux de ses fragments contre lesquels sont dirigés les anticorps mis en oeuvre ;
- des doses appropriées d'anticorps anti-immunoglobulines de lapin (ou d'un autre mammifère approprié) ;
- un système enzymatique (tel que PAP-système peroxydase-antiperoxydase notamment), de révélation de la réaction anticorps-antigène ;
- un substrat pour la révélation de la réaction enzymatique, tel que, notamment le DAB ;
- éventuellement une substance de coloration des cellules tumorales, si le prélèvement biologique est constitué par des coupes histologiques et si la coloration de celles-ci est désirée ;
- des quantités utiles de tampons appropriés pour la mise en oeuvre du test de dépistage et de diagnostic.

On peut, bien entendu, mettre en oeuvre tout autre système de marquage et de révélation approprié de l'anticorps, par une méthode directe ou indirecte (notamment par la méthode sandwich, par les systèmes de marquage à l'avidine et à la biotine, par fluorescence, phosphorescence, protéine A, RIA, ELISA, etc...).

Bien que l'hormono-dépendance de certains cancers du sein soit bien connue, la plupart des patientes présentant des tumeurs contenant des RE (récepteurs d'oestrogènes), mais pas toutes, peuvent tirer bénéfice d'un traitement hormonal. D'un autre côté, le fait que 30 à 40% des patientes présentant des cancers riches en RE répondent négativement à un traitement hormonal a été attribué soit à l'hétérogénéité de la tumeur, soit au fait que les RE pouvaient ne pas être fonctionnels. Il a été, en outre, suggéré que la détermination de la teneur en RP (récepteurs de progestérone), dont l'expression est connue comme étant oestrogène-dépendante dans les tissus reproducteurs normaux et dans la lignée cellulaire MCF-7 dérivée d'un cancer du sein, pourrait permettre l'identification de tumeurs présentant des récepteurs fonctionnels. En fait, tous les cancers du sein RE(+) et RP(+) ne répondent pas à l'hormono-thérapie (seulement 80% le font), ce qui montre la nécessité de disposer de marqueurs additionnels de réponse à l'hormonothérapie pour permettre une identification plus aisée des cancers du sein qui pourraient bénéficier d'une hormonothérapie.

Selon un mode de réalisation avantageux de la méthode de détection et de diagnostic conforme à l'invention, des prélèvements biologiques utilisés pour la détection d'une tumeur du sein sont soumis à une détection immunologique simultanée des RE, des RP et de la protéine pS2 ou d'un de ses fragments, faisant apparaître d'une part, une hétérogénéité fonctionnelle additionnelle dans les cancers du sein RE(+) (avec 60% environ seulement de tumeurs RE(+) également positives pour les Rp et l'expression du gène pS2, et d'autre part, une grande homogénéité des cancers RE(-) en ce qui concerne les Rp et l'expression du gène pS2 (avec environ 96% d'absence d'expression de ces deux gènes inductibles par des oestrogènes).

Selon une disposition avantageuse de ce mode de réalisation, les tests de détection immunologique des RE et des RP sont des tests immunocytochimiques réalisés sur des coupes histologiques congelées à -180°C, de préférence dans l'isopentane.

Les techniques de mise en oeuvre pour la détection immunologique des RE et des RP sont connues et décrites dans la littérature.

Selon un autre mode de mise en euvre de l'invention, on effectue le dépistage et le diagnostic d'états pathologiques de l'estomac, notamment des cancers et des ulcères.

En effet, les Inventeurs ont mis en évidence la présence, dans des tumeurs de cancers de l'estomac, du peptide de séquence (D).

Cette observation permet notamment de dépister et de diagnostiquer des cancers de l'estomac et de déterminer l'origine de métastases.

Conformément à l'invention la méthode de dépistage et de diagnostic s'applique à la détermination in vivo par immunoscintigraphie à l'aide des anticorps conformes à l'invention, d'états pathologiques d'un organe.

La présente invention a également pour objet des agents thérapeutiques, caractérisés en ce qu'ils contiennent un ou des anticorps conformes à la présente invention.

Conformément à l'invention lesdits anticorps peuvent être associés à un autre agent thérapeutique.

La présente invention permet de réaliser un dosage quantitatif de la protéine pS2 grâce à l'obtention d'anticorps monoclonaux dirigés contre le peptide de synthèse D, ainsi que contre la protéine native extraite de différents milieux, conformément à la présente invention.

Outre les dispositions qui précèdent, l'invention comprend encore d'autres dispositions, qui ressortiront de la description qui va suivre.

L'invention sera mieux comprise à l'aide du complément de description qui va suivre, qui se réfère à des exemples de réalisation des objets de l'invention, dont ils ne constituent en aucune manière une limitation.

Il doit être bien entendu, toutefois, que ces exemples de réalisation sont donnés uniquement à titre d'illustration des objets de l'invention, dont ils ne constituent en aucune manière une limitation.

**Exemple 1** - Purification du peptide (D) produit par les cellules MCF-7.

On cultive des cellules MCF-7 en présence d'oestradiol $10^{-9}$ M pendant 4 jours.

On recueille le surnageant de culture enrichi en peptide (D) sécrété dans le milieu de culture par lesdites cellules et on le soumet successivement à l'action :
- de l'acétone (IV) à -20°C pour précipiter des protéines de haut poids moléculaire,
- de l'HCl 12N (à pH 1 environ) pour précipiter les protéines insolubles à ce pH.

On centrifuge successivement les deux précipités pour éliminer les culots et ne conserver que les surnageants renfermant le peptide (D). Ce surnageant est traité par 4 volumes d'acétone à -20°C pour précipiter les autres protéines présentes, dont le peptide (D). On centrifuge ; on élimine le surnageant ; on recueille le culot qui renferme le peptide (D).

Le culot est lyophilisé ; on ajoute au lyophilisat 3 ml de tampon KCl, 50 mM EDTA ; on le soumet à une chromatographie en HPLC sur G 3000 et phase inverse successivement.

On récupère 120 fractions de 1,5 ml chacune qui sont soumises à un Western blotting (immuno-empreinte). Les fractions contenant le peptide (D) sont identifiées à l'aide d'anticorps polyclonal de lapin dirigé contre un peptide de synthèse correspondant aux 31 amino-acides C-terminaux du peptide (D).

Les fractions positives sont réunies et soumises à une deuxième chromatographie en HPLC phase inverse de type BROWNLEE-AQUAPORE RP 300, puis congelées à -20°C ou concentrées par centrifugation sous vide.

L'échantillon est contrôlé sur gel SDS-PAGE, révélé par coloration des protéines au nitrate d'argent.

On isole le peptide (D) pur à 80%.

**Exemple 2** - Purification du peptide (D) sécrété par les cellules à mucus de l'estomac à partir de suc gastrique.

On recueille du suc gastrique d'individus à jeun (pH1) que l'on neutralise par du tampon carbonate/bicarbonate à pH 9,6 et que l'on centrifuge afin d'éliminer les débris.

On soumet le surnageant à l'action :
- de l'acétone à -20°C à raison de 4 volumes d'acétone par volume de surnageant.

On centrifuge, on élimine le surnageant, et on recueille le culot, qui contient le peptide (D), et que l'on lyophilise.

On soumet ce culot à une chromatographie en HPLC sur G3000. Les fractions récupérées sont identifiées par Western blotting et les fractions positives sont réunies et concentrées par centrifugation sous vide. On les soumet ensuite à une deuxième chromatographie en HPLC phase inverse de type BROWNLEE-AQUAPORE RP 300 et on identifie les fractions positives par Dot-Blot, qui sont reconcentrées par centrifugation sous vide.

**Exemple 3** - Synthèse de la protéine de formule I et de ses fragments, notamment des peptides de formules (C), (D), (E), (F).

La synthèse de chacune de ces substances est réalisée en mettant en oeuvre la méthode en phase solide de MERRIFIELD. Après lyophilisation, la protéine ou les peptides synthétisés sont respectivement réduits en présence d'un mélange d'urée/β-mercaptoéthanol, relargués pour en éliminer les sels, relyophilisés et leur composition en amino-acides est vérifiée.

**Exemple 4** - Préparation d'anticorps polyclonaux à l'aide du peptide de formule (C)

Le peptide (C) composé des 31 amino-acides à l'extrémité carboxy-terminale de la protéine (I), synthétisé conformément à l'Exemple 3, est injecté par voie sous-cutanée à des lapins, 3 fois à 2 semaines d'intervalle, à raison de 200 μg, mélangé à de l'adjuvant complet de Freund pour les deux premières injections. Le sérum a été collecté deux semaines après la dernière injection et utilisé à une dilution appropriée.

Selon la même méthode, on prépare des anticorps polyclonaux à partir de tous les fragments de la protéine (I) et notamment à partir des peptides de formules (D), (E), (F).

**Exemple 5** - Préparation d'anticorps monoclonaux spécifiques

A) Immunisation des souris avec le peptide de synthèse (F)

Des souris Biozzi ont été immunisées avec le peptide de synthèse de séquence (F) qui correspond aux 28 acides aminés de l'extrémité C-terminale de la protéine pS2.

Les deux premières injections ont été réalisées avec le peptide (F) (100 μg) couplé à de l'ovalbumine au moyen de la glutaraldéhyde (F-OVA néosystem laboratoire). Le taux de couplage obtenu est de 25 moles de peptide (F) pour 1 mole d'ovalbumine. Les injections ont ensuite été poursuivies avec le peptide (F) (100 μg) seul. Les injections ont été effectuées tous les 15 jours et ont toutes été faites en intrapéritonéale avec adjonction d'adjuvant de Freund complet pour la première, et adjonction d'adjuvant incomplet de Freund pour la deuxième et la troisième injections. 10 jours après la troisième injection, on prélève une goutte de sang à l'oeil de l'animal et le sérum obtenu après coagulation est testé par RIA contre le peptide (D) purifié et iodé [voir description détaillée de la méthode de détection par RIA dans le présent Exemple 5 chapitre C) ci-dessous]. Si le titre de sérum est correct, la souris est réinjectée deux fois, deux jours et quatre jours avant la fusion.

Si le titre du sérum n'est pas correct, on refait des injections à la souris toutes les trois semaines jusqu'à ce que le test soit positif.

B) Obtention des hybridomes

La fusion a été réalisée en présence de PEG avec des cellules de myélome X63, à raison de deux cellules de rate pour une cellule de myélome. Les hybridomes obtenus ont été distribués en 3 plaques de 24 puits. L'addition de HAT dans le milieu de culture quatre heures après la fusion permet de sélectionner les hybridomes. Les cultures ont été poursuivies en présence de macrophages. Les premiers tests de surnageant de culture ont été entrepris environ 8 jours après la fusion. Lesdits tests sont identiques à ceux effectués sur les sérums, à savoir par technique RIA et contre la protéine native [voir description détaillée de la méthode de détection par RIA dans le présent Exemple 5 chapitre C) ci-dessous]. La classe des anticorps produits a également été déterminée par technique ELISA en utilisant des seconds anticorps anti sous-classe stricte.

13 puits renfermant un ou plusieurs hybridomes ont ainsi été sélectionnés : 4 d'entre eux ont été clonés : CIB3, CIB6, CIC4, CIIID5. A la suite du premier clonage, les clones CIC4-12 ainsi que CIIID5-20, tous deux positifs en RIA ont été clonés une deuxième fois. A l'issue de ce deuxième clonage, 10 des 12 colonies repiquées à partir du CIIID5-20 renfermaient l'anticorps anti-peptide (F). Parmi eux, le CIIID5-20-9($p28_1O_2$) a été choisi sur des critères de culture. L'autre clonage ne mettant en évidence que 3 clones positifs sur 12, un troisième clonage a été effectué où 11 colonies sur 12 se sont révélées positives. Comme précédemment, l'une d'entre elles : CIC4-12-4-7($p28_2O_3$), a été choisie.

Certains des clones obtenus permettent une excellente détection immunohistochimique du peptide de séquence (D) sur les coupes de biopsies de cancers du sein, en tous points identiques à celle précédemment obtenue avec les anticorps polyclonaux dirigés contre les peptides de synthèse et notamment le peptide (C).

C) Test RIA (Radio Immuno Assay) de détection des anticorps dirigés contre le peptide (D) mis en oeuvre aux étapes A) et B) ci-dessus

Le test RIA utilisé pour la vérification de la présence du peptide (D) et de la sélection des hybridomes a été réalisé comme suit :

Le peptide (D) a été extrait d'un milieu de culture MCF7 et a été ensuite partiellement purifié par chromatographies HPLC successives. On obtient ainsi une fraction renfermant 80% de peptide (D). Cette fraction enrichie est ensuite marquée à l'iode 125 (ORIS Lapam) par la méthode à la chloramine T. Le rendement de marquage est de l'ordre de 2 000 Ci/m mole.

Le peptide (D) iodé est alors séparé de l'iode libre par passage sur une colonne d'affinité réalisée par couplage d'un anticorps polyclonal de lapin sur du sépharose CN4B. On récupère le peptide par décrochage en milieu HCl Glycine pH 2,8. Il est ensuite aliquoté et prêt à l'emploi.

Le test consiste en l'addition de :

200 µl de PBS BSA O,3%

100 µl de Traceur $^{125}$1 (D) (environ 30 000cpm)

100 µl de Milieu renfermant l'anticorps à tester

On agite et on laisse durant une nuit à température ambiante.

Le lendemain, on ajoute :

50 µl de NHS (sérum normal humain)

50 µl de SMAS (sérum de mouton antisouris)

On agite et on laisse 15 minutes à température ambiante.

On centrifuge 15 minutes à 3000 t/mn (Jouan) et on compte les culots dans un compteur gamma.

Pour chaque série de tests, on effectue :

- un tube (T) renfermant seulement du peptide iodé, cela donne le maximum de cpm pour la quantité utilisée (environ 30 000 cpm) ;
- un tube (NBS) pour lequel on n'ajoute pas de solution d'anticorps anti-D, ce qui donne le bruit de fond de la manipulation (environ 600 cpm). Les sérums à tester sont dilués au 1/100e et 1/1000e.

Exemple 6 - Caractérisation du peptide de séquence (D).

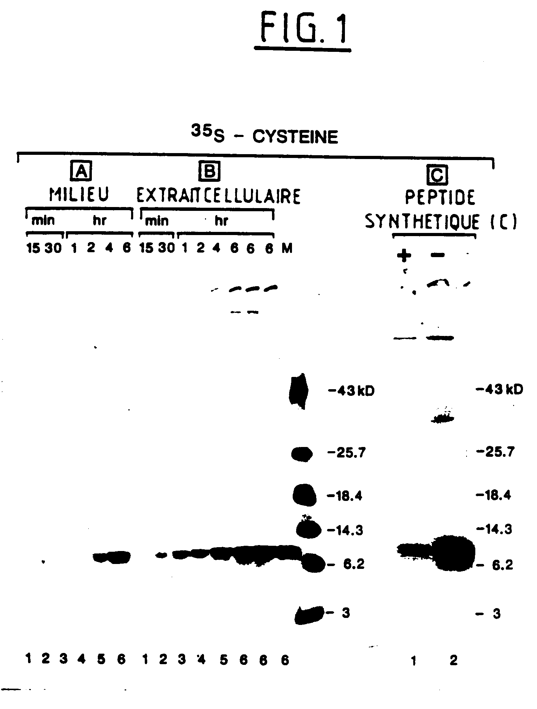

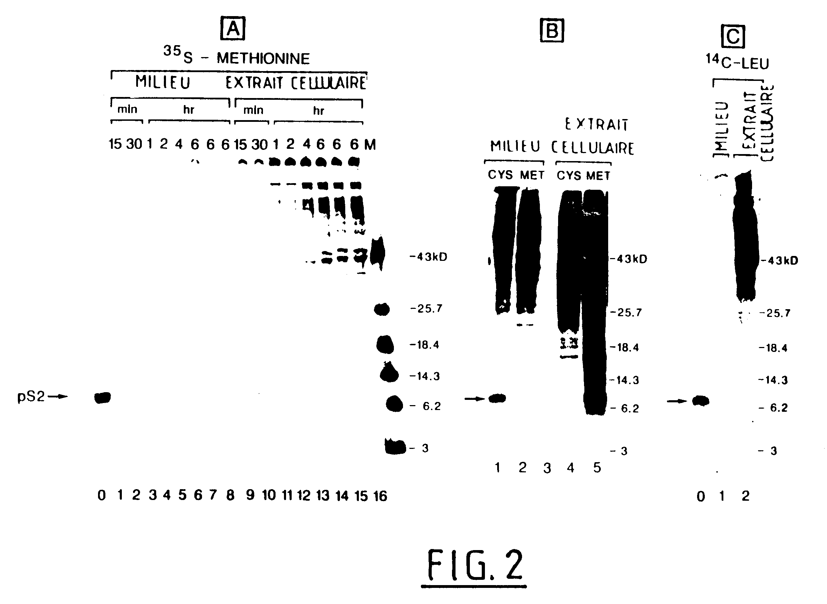

70 à 90% de monocouches confluentes de cellules MCF-7 cultivées en présence d'oestradiol ont été marquées par de la $^{35}$S-cystéine (cf. figure 1 annexée) ou de la $^{35}$S-méthionine (cf. figure 2 annexée) et le milieu ainsi que des extraits cellulaires ont été collectés à des intervalles déterminés. Des aliquots ont été immunoprécipités par de l'anti-sérum pS2 et analysés sur un gradient de 15 à 25 % de gel de SDS-PAGE. Le déroulement dans le temps relatif à la $^{35}$S-cystéine (figure 1, A et B) montre la présence d'une seule bande située à environ 7 KD. Dans l'extrait cellulaire (partie B de la figure 1) le signal est déjà visible au bout de 15 minutes de marquage. Lorsque les échantillons de milieu correspondant à des aliquots identiques du milieu de culture total ont été analysés (partie A de la figure 1), le signal est devenu visible au bout d'une heure de marquage, ce qui suggère un certain retard dans le processus de sécrétion. Dans la figure 1 C,l'immunoprécipitation du peptide (D) dans le milieu (colonne 2) est montrée comme étant spécifique attendu que la bande du peptide (D) est en compétition avec le peptide synthétique C (colonne 1).

Contrairement à cela, aucune bande de la dimension attendue pour le peptide (D) n'a pu être détectée à aucun moment du déroulement dans le temps, aussi bien dans le milieu que dans l'extrait cellulaire après marquage par la $^{35}$S-méthionine et immunoprécipitation par l'anti-sérum (cf. figure 2, partie (A)). Le schéma de bande présent dans la partie supérieure du gel a persisté après utilisation d'un sérum témoin non-immun (colonnes 7 et 15) et il n'est pas entré en compétition par l'addition du peptide synthétique (C) à la réaction d'immunoprécipitation (colonnes 8 et 16), ce qui indique qu'il correspond à une adsorption non spécifique.

Le milieu de culture et les extraits cellulaires ont également été analysés sans immunoprécipitation postérieure au marquage par la $^{35}$S-cystéine (cf. figure 2b, colonnes 1 et 4) ou par la $^{35}$S-méthionine (colonne 5). Une bande (fléchée), marquée avec de la cystéine (colonne 1) mais pas par de la méthionine (colonne 2) était clairement visible dans le milieu de culture et elle migrait au même niveau que le peptide (D) immunoprécipité (colonne 3). Contrairement à cela, il n'y a pas eu de marquage différentiel dans l'extrait cellulaire total, ce qui indique que la masse des protéines qui migre à ce niveau ne correspond pas au peptide (D).

Les résultats qui viennent d'être mentionnés fondent l'hypothèse selon laquelle les trois résidus méthionine placés à l'extrémité amino-terminale de la séquence de la protéine pS2 déduite de l'ADNc, appartiennent à un peptide signal qui est rapidement clivé.

La figure 1 représente le déroulement dans le temps du marquage du peptide (D) par la $^{35}$S-cystéine. Les parties (A) et (B) montrent respectivement des cellules MCF-7 cultivées en présence d'oestradiol et de rouge

EP 0 345 315 B1

de phénol marquées pour des durées différentes par la $^{35}$S-cystéine : 15 minutes, 30 minutes, 1 heure, 2 heures, 4 heures et 6 heures, dans les colonnes 1 à 6 respectivement. Des aliquots du milieu (200 μl) (partie A) et des extraits cellulaires (25 μl) (partie B) correspondant à des fractions identiques de la culture totale ont été immunoprécipités puis analysés sur un gradient de 15 à 25 % de gel de SDS-PAGE et par fluorographe. M désigne des marqueurs de poids moléculaire. La partie C représente un marquage de 16 heures par la $^{35}$S-cystéine ; colonne 2 l'immunoprécipité d'un aliquot de milieu ; colonne 1 : même chose que colonne 2 mais en présence du peptide synthétique compétiteur (C.)

Figure 2, partie A, tentative de marquage du peptide (D) par de la $^{35}$S-méthionine dans le milieu (colonnes 1 à 8) et l'extrait cellulaire (colonnes 9 à 16). Même expérience que dans la Figure 1, mais en présence de $^{35}$S-méthionine. La colonne 0 est identique à la colonne 5 de la figure 1 A, la durée du marquage a été de 15 minutes, 30 minutes, 1 heure. 2 heures, 3 heures, 4 heures et 6 heures comme indiqué. L'immunoprécipitation a été effectuée avec du sérum non-immun (colonnes 7 pour le milieu et 15 pour l'extrait cellulaire) ou en présence de sérum immun et du peptide synthétique compétiteur (C) (colonnes 8 pour le milieu et 16 pour l'extrait cellulaire). M désigne des marqueurs de poids moléculaire.

Partie B : le peptide (D) peut être détecté directement dans le milieu. La synthèse de la protéine par les cellules MCF-7 a été réalisée en présence de $^{35}$S-cystéine (colonnes 1 et 4) ou de $^{35}$S-méthionine (colonnes 2 et 5). Les cellules ont été cultivées en présence de rouge de phénol et d'oestradiol et marquées pour une durée de 6 heures. Des aliquots de milieu et d'extraits cellulaires contenant le même nombre de comptages précipitables de TCA ont été analysés directement sur un gradient de 15 à 25% de gel de SDS-PAGE. Colonnes 1 et 2 : milieu de culture ; colonnes 4 et 5 : extraits cellulaires. Colonne 3 et la flèche : protéine pS2 immunoprécipitée, marquée in vivo par la $^{35}$S-cystéine.

Partie C : tentative de marquer le peptide (D) par de la $^{14}$C-leucine. Des cellules MCF-7 cultivées en présence d'oestradiol et de rouge de phénol ont été marquées pour une durée de 6 heures par de la $^{14}$C-leucine. Des aliquots de 200 μl de milieu de culture (colonne 2) ou d'extraits cellulaires (colonne 3) ont été immunoprécipités comme dans les figures 1 et 2a. La colonne 1 et la flèche donnent la position du peptide (D) immunoprécipitée marquée in vivo par de la $^{35}$S-cystéine. Exposition du film : deux mois.

**Exemple 7** - Séquençage du peptide (D) isolé des cellules MCF- 7 (pS2M)

Après réduction et alkylation des cystéines sous forme de S-β-(4-pyridylethyl)-cystéine par la méthode de FRIEDMANN puis dégradation par la trypsine on obtient 6 peptides majeurs qui sont séparés par chromatographie sur phase inverse C8.

Les cystéines sont distribuées dans la protéine de telle manière que chaque peptide obtenu par dégradation trypsique contienne au moins un S-β-(4-pyridyléthyl)-cystéine, dosée à une longueur d'onde de 254 nm.

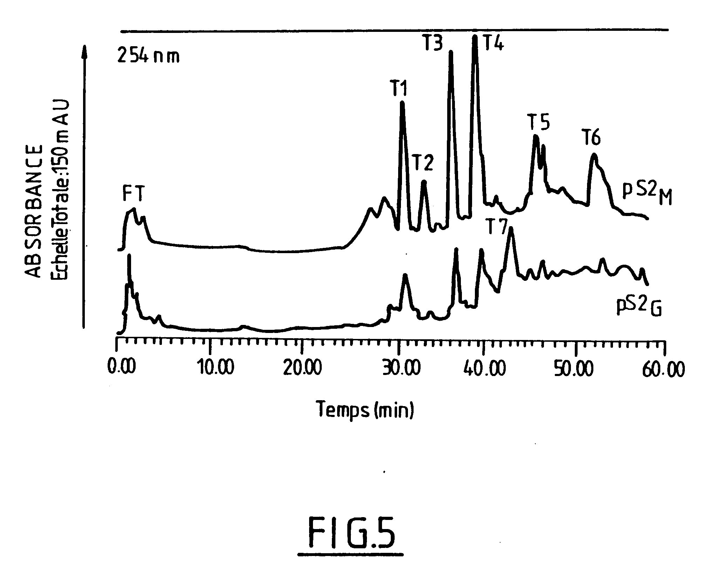

On obtient 6 fragments trypsiques nommés T1 à T6 (figure 5. courbe supérieure).

La figure 5 représente le fractionnement des peptides trysiques du peptide (D) isolé de suc gastrique (pS2G) et des cellules MCF-7 (pS2M), portant des résidus S-β-(4-pyridyléthyl)-cystéine, par chromatographie sur colonne microbore Aquapore Brownlee RP300 (7μC8/2, 1 x30mn), équilibrée par un tampon composé d'une solution à 0,1% de TFA (acide trifluoroacétique pH2). Les colonnes sont éluées en 60 minutes par un gradient de 0 à 80% en tampon B qui est composé d'acétonitrile renfermant 0,1% de TFA. Le débit de la colonne est de 0,1 ml/min. et les différents peptides sont détectés par leur absorbance à 254 nm en fonction de leur temps d'apparition.

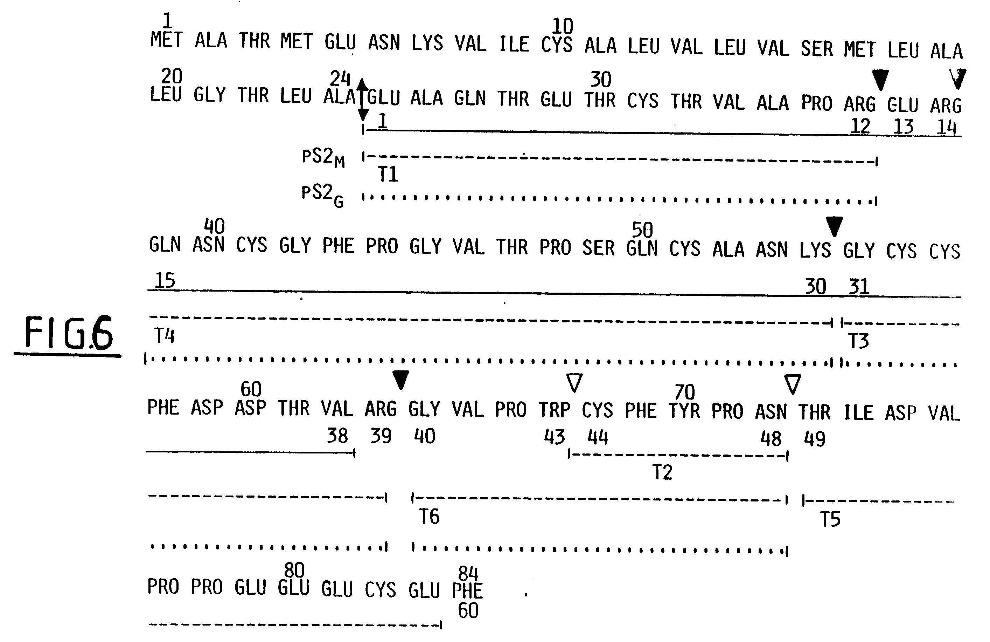

Ces peptides trypsiques ont été séquencés et on obtient la carte du peptide (D) isolé de cellules MCF-7 (figure 6).

La figure 6 représente la séquence de la protéine pS2 en indiquant les sites de coupure par le signal peptidase ( $\updownarrow$ ) , qui donne naissance au peptide (D), par la trypsine (▲), et les sites de coupure qui ne sont pas imputés à ces deux enzymes (Δ).

La séquence de ce peptide permet de définir quatre sites de coupure par la trypsine situés après les acides aminés basiques $Arg_{36}$, $Arg_{38}$, $lys_{54}$ et $Arg_{63}$.

L'action de la trypsine aurait donc dû générer les 5 peptides suivants : $GLU_{25}$ - $ARG_{36}$, $GLU_{37}$ - $ARG_{38}$, $GLN_{39}$ - $LYS_{54}$, $GLY_{55}$ - $ARG_{63}$ et $GLY_{64}$ - $PHE_{84}$. En fait parmi les 6 pics observés seuls T1, T3 et T4 correspondent à des peptides trypsiques prévus respectivement $GLU_{25}$ - $ARG_{36}$, $GLY_{55}$ - $ARG_{63}$ et $GLN_{39}$ - $LYS_{54}$.

Les autres pics (T2, T5 et T6) proviennent de coupures inattendue :
- la coupure entre $TRP_{67}$ et $CYS_{68}$ ne concerne que 30% des molécules pS2M et provient d'une contamination chymotrysique de la préparation de Trypsine.
- les coupures entre $ASN_{72}$ et $THR_{73}$ ne proviennent ni de la trypsine, ni de la chimotrypsine et sont l'oeuvre d'une peptidase soit cosécrétée, soit contaminant la trysine.

En outre le dipeptide $GLU_{37}$ $ARG_{38}$ trop petit pour être retenu sur la colonne n'est donc pas détecté, et sa présence a été déduite de la séquence de la protéine non modifiée.

La comparaison de la séquence obtenue en mettant bout à bout ces peptides trypsiques, et de la séquence de la protéine non modifiée de 84 acides aminés montre que la signal peptidase coupe la protéine initiale après le résidu ALA situé en position 20 après la méthionine N-terminale. La forme sécrétée commence donc par la séquence GLU - ALA - GLN.

**Exemple 8** - Séquençage du peptide (D) isolé du suc gastrique (pS2G)

Le protocole de réduction et d'alkylation des cystéines puis de trypsino-lyse utilisé pour séquencer le peptide (D), isolé du suc gastrique (pS2G), est le même que celui utilisé pour séquencer pS2M.

La chromatographie du lysat obtenu présente un tracé présentant quelques différences comparativement à celui obtenu dans le cas de pS2M.
- les fragments T1, T3 et T4 et T6 ont été séquencés et sont identiques dans les deux cas
- les séquences correspondant à T2 et T5 n'ont pas été retrouvées
- aucune séquence n'a pu être obtenue à partir du pic supplémentaire T7.

L'absence du peptide T2, comparativement à la préparation de pS2M, n'a en soi rien de surprenant, ce peptide résultant de la contamination.

Le site de coupure de la signal peptidase est donc identique pour le peptide isolé de deux types cellulaires différents.

On observe une différence entre pS2M et pS2G en ce qui concerne le résidu GLU situé à l'extrémité N-terminale de la protéine. En effet, dans le cas de pS2G cet acide aminé est cyclisé sous la forme d'un pyro-GLU, ce qui empêche d'établir la séquence à partir de la forme non modifiée. Cependant le peptide trypsique N-terminal a pu être séquencé, ce qui indique un débloquage de cette extrémité N-terminale.

**Exemple 9** - Détection immunocytochimique du peptide (D)

1) Préparation de coupes histologiques.

Un échantillon chirurgical a été découpé en fines lamelles qui ont été utilisées pour la détection immunocytochimique des RE, des RP et du peptide (D). Les échantillons prévus pour la détection immunocytochimique des RE et des RP ont été congelés dans de l'isopentane à - 180°C, tandis que les échantillons prévus pour la détection immunocytochimique du peptide (D) ont été enrobés dans de la paraffine et fixés par de la formaline à 10% pendant 24 heures.

2) Coloration immunocytochimique du peptide (D).

Les échantillons enrobés de paraffine et fixés à la formaline ont été découpés, débarrassés de la paraffine à l'aide de toluène et rincés parfaitement à l'éthanol absolu. Après lavage à l'eau et au PBS, les coupes ont été incubées avec du sérum de mouton (2,5% dans du PBS contenant 0,5% de gammaglobulines bovines) pour réduire les colorations non spécifiques. Après incubation avec des anticorps polyclonaux de lapin anti-peptide (à une concentration de 1:640) les coupes ont été incubées successivement avec des IgG de mouton anti-lapin, puis avec un système péroxydase anti-péroxydase de lapin à une concentration de 1:10 pour les IgG, et de 1:50 pour le système péroxydase anti-péroxydase. Chaque incubation a été suivie d'un lavage au PBS. Après un rinçage final par le PBS, les coupes ont été incubées avec du DAB et colorées à l'hématoxyline à une concentration de 1:10. Les déterminations immunocytochimiques des RE et des RP ont été réalisées sur des coupes congelées en utilisant des kits d'anticorps monoclonaux fournis par Abbot et Transbio respectivement, conformément à la procédure recommandée par ces fournisseurs. L'intensité de la coloration a été estimée sur une échelle à 4 points (0 à 3+) et la proportion de cellules tumorales colorées positivement a été déterminée en pourcentage des cellules tumorales totales (1 à 100%) ; l'indice de coloration a été obtenu en multipliant le pourcentage de cellules colorées par le score de coloration (1 à 300).

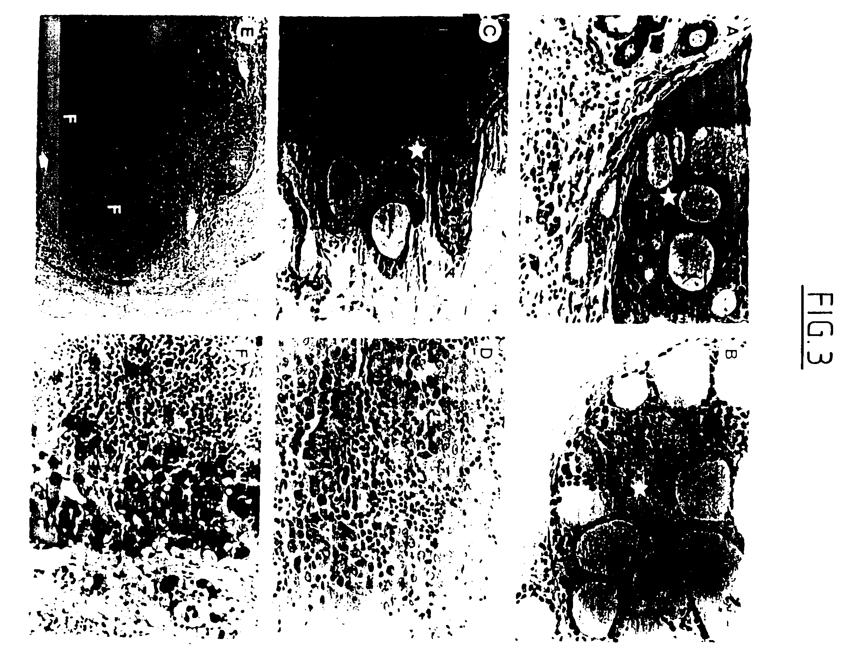

3) Le test de coloration du peptide (D) s'est avéré être cytoplasmique aussi bien sur des coupes de cellules cancéreuses de sein que sur des coupes de nodules métastasiques (cf. figure 3, parties A, E, F). Toutefois, la répartition cytoplasmique de la coloration n'était pas uniforme avec, fréquemment, une condensation périnucléaire (cf. flèche à la figure 3, partie F) qui peut correspondre à l'appareil de GOLGI, car comme on le sait, le peptide (D) est sécrété. En outre, l'intensité de la coloration s'est avérée variable d'une cellule à une autre, même à l'intérieur d'une surface donnée d'une coupe (cf. figure 3A et F). Il en a également été de même pour la coloration nucléaire spécifique des RE (figure 3B). Toutefois, tous les carcinomes de sein ne se sont pas avérés positifs vis-à-vis de la coloration spécifique du peptide (D) (figure 3D). La coloration s'est avérée spécifique pour le peptide (D) comme l'a démontré sa suppression lorsqu'on l'a mis en compétition avec l'un des peptides synthétiques utilisé pour réaliser les anticorps qui, dans le cas présent a été le peptide (C) comprenant 31 acides aminés (figure 3, partie C). En outre, des cellules d'épithélium ductulaire normal ("N" à la figure 3) et des tumeurs bénignes de sein n'ont pas été colorées.

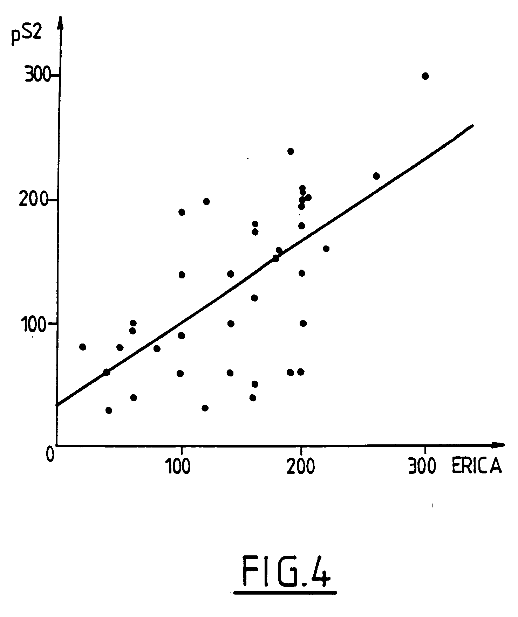

Une bonne corrélation a été observée entre l'indice de coloration du peptide (D) et le score d'intensité de l'ARNm pS2 déterminé à partir de l'analyse par NORTHERN-blot d'ARN décrite précédemment dans la littérature, ce qui indique que la présence du peptide (D) reflète très probablement une transcription augmentée, ainsi qu'il a déjà été démontré précédemment dans la littérature en utilisant la lignée cellulaire de cancer du sein MCF-7. Le fait que cette induction soit RE-indépendante est fortement supporté par la bonne corrélation qui existe entre l'indice de coloration du peptide (D) et l'indice de coloration des RE (cf. figure 4 et Tableau I annexés). On peut en conclure que l'expression du gène pS2 dans les cancers du sein est très probablement oestrogène-dépendante comme c'est le cas dans la lignée cellulaire MCF-7

**Exemple 10** - Expression comparative du gène pS2 avec ceux des récepteurs d'oestrogène et de progestérone et l'oncogène erbB-2.

Le Tableau 1 annexé constitue un résumé des résultats qui ont été obtenus pour 180 échantillons de cancer du sein pour lesquels tous les paramètres ont été déterminés de façon non équivoque à peu d'exceptions près en ce qui concerne l'expression des RE (récepteurs d'oestrogène), des RP (récepteurs de progestérone), du gène pS2 (pS2) et de l'oncogène erbB-2. Les taux de RE et de RP ont été déterminés en utilisant à la fois les tests RE-DCC, RP-DCC, RE-ICA et RP-ICA sauf pour 2 des 7 cas "RE(+),RP(-) et pS2(+)" pour lequels les RP n'ont été testés que par le test RP-DCC /DCC = "dextran-coated charcoal stéroïde binding assay" (test de fixation des stéroïdes par le charbon dextran) ; ICA = test immunocytochimique/. L'expression du gène pS2 a été déterminée en utilisant aussi bien l'analyse à l'aide d'ARNm pS2; que le test pS2-ICA. L'excès d'expression (positivité) et les taux d'expression non détectables ou très faibles (négativité) du gène erbB-2 ont été déterminés par analyse à l'aide d'ARNm. La répartition des métastases des nodules lymphatiques axillaires (ALN) est indiquée dans la dernière colonne de droite du Tableau I. RE(+) et RP(+) correspondent à des échantillons qui se sont avérés positifs en utilisant au moins l'un des tests RE et RP ; pS2(+) correspond à des échantillons positifs pour des tests utilisant l'ARNm pS2 et/ou le test immunocytochimique pour le pS2 (et habituellement pour les deux) et un excès d'expression pour erbB-2-(+) a été déterminé par analyse à l'aide d'ARNm.

## TABLEAU I

EXPRESSION DE pS2, erbB-2, RE et RP dans 180 BIOPSIES DE CANCER
DU SEIN

ALN(+) indique la présence d'au moins un nodule métastasé.

Les pourcentages indiqués entre parenthèses se réfèrent au nombre total de tumeurs (180 cas), tandis que les pourcentages entre crochets se réfèrent soit à des tumeurs RE(+) (129 cas, partie supérieure du Tableau I), soit à des tumeurs RE(-) (51 cas, partie inférieure du Tableau I).

Tous les cancers du sein peuvent être classés en six sous-classes d'importance inégale, comme le montre le Tableau I. 72% des tumeurs sont RE(+), dont 88% sont RP(+) et 12% RP(-). Cependant, tous les cancers RP(+) ne sont pas pS2(+) et tous les cancers RP(-) ne sont pas pS2(-). En fait, seulement 62% des tumeurs RE(+) se sont avérées également positives pour l'expression de RP et de pS2, alors que 26% des tumeurs RE(+) se sont avérées RP(+) et pS2(-). Il s'est également avéré que quelques tumeurs "RE(+), RP(-)" étaient soit pS2(+), soit pS2(-). Ces observations présentent un grand intérêt attendu que la transcription aussi bien du gène RP que du gène pS2 peut être induite par un oestrogène dans les cellules MCF-7. Ainsi, la détermination simultanée de l'expression des gènes RE, RP et pS2 fait apparaître une hétérogénéité fonctionnelle additionnelle dans les cancers du sein RE(+). Ceci peut refléter soit une hétérogénéité des RE eux-mêmes, qui serait apte à induire aussi bien les gènes RP que le gène pS2, ou aucun d'entre eux, ou bien une hétérogénéité dans l'aptitude à la réponse aux oestrogènes, des gènes RP et pS2. D'autre part, les cancers RE(-) apparaissent très homogènes en ce qui concerne l'expression de RP et de pS2, attendu que 96% d'entre eux n'expriment aucun de ces deux gènes inductibles par un oestrogène. L'excès d'expression erbB-2 a été également réparti entre les diverses sous-classes. Il est à souligner que la détermination par voie cytoimmunochimique des RE, des RP et du peptide (D) présente une grande utilité pour l'examen de l'hétérogénéité des tumeurs en ce qui concerne leur réponse vis-à-vis des oestrogènes et, par voie de conséquence, l'aptitude au traitement des cancers du sein par hormono-thérapie.

**Exemple 11** - Dosage du peptide (D) sur du cytosol.

Le cytosol est préparé à partir de prélèvements effectués sur des tumeurs mammaires, par bioponction ou par broyage de tissus, les prélèvements étant congelés dans du tampon Tris 10 mM contenant de l'EDTA 1,5 mM , du monothioglycérol 10 mM, du molybdate de sodium 10 mM, pH 7,4, tel que décrit pour les dosages de récepteurs hormonaux, ce qui évite une préparation spécifique du cytosol, et permet de doser le peptide (D) en plus des récepteurs déjà dosés en routine.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière sans s'écarter du cadre, ni de la portée, de la présente invention.

**Revendications**

1. Peptide, caractérisé en ce qu'il est identifié comme étant la forme sécrétée de la protéine pS2, en ce qu'il est constitué par un fragment correspondant à 60 acides aminés de ladite protéine pS2, en ce qu'il commence par la séquence GLU - ALA - GLN, GLU étant situé en vingt cinquième position après la MET N-terminale de la protéine pS2 et en ce que son poids moléculaire est de 6 600 Daltons environ, ledit peptide étant dit "peptide pS2 ".

2. Peptide selon la revendication 1, caractérisé en ce que sa séquence (D) en acides aminés est la suivante :

```
                         30
GLU  ALA  GLN  THR  GLU  THR  CYS  THR  VAL  ALA  PRO  ARG  GLU  ARG
     40
GLN  ASN  CYS  GLY  PHE  PRO  GLY  VAL  THR  PRO  SER  GLN  CYS  ALA
                                   60
ASN  LYS  GLY  CYS  CYS  PHE  ASP  ASP  THR  VAL  ARG  GLY  VAL  PRO
               70                                            80
TRP  CYS  PHE  TYR  PRO  ASN  THR  ILE  ASP  VAL  PRO  PRO  GLU  GLU
GLU  CYS  GLU  PHE

                              (D)
```

3. Peptide selon l'une quelconque des revendications 1 et 2, caractérisé en ce qu'il comprend quatre sites de coupure par la trypsine, qui sont situés après les acides aminés basiques $Arg_{36}$, $Arg_{38}$, $Lys_{54}$ et $Arg_{63}$.

4. Peptide caractérisé en ce qu'il correspond aux 12 acides aminés N-terminaux du peptide de séquence (D) selon la revendication 2.

5. Peptide caractérisé en ce qu'il correspond à la séquence d'acides aminés allant de la $GLN_{39}$ à la $LYS_{54}$ du peptide de séquence (D) selon la revendication 2.

6. Peptide caractérisé en ce qu'il correspond à la séquence d'acides aminés allant de la $GLY_{55}$ à l'$ARG_{63}$ du peptide de séquence (D) selon la revendication 2.

7. Peptide caractérisé en ce qu'il correspond aux 21 acides aminés C-terminaux du peptide de séquence (D) selon la revendication 2.

8. Peptide caractérisé en ce qu'il est constitué par un fragment correspondant aux 31 acides aminés C-terminaux du peptide pS2 selon la revendication 2 et en ce qu'il est d'un poids moléculaire d'environ 3450 Da.

9. Peptide caractérisé en ce qu'il est constitué par un fragment correspondant aux 30 acides aminés N-terminaux du peptide pS2 selon la revendication 2 et en ce qu'il est d'un poids moléculaire d'environ 3300 Da.

10. Peptide selon la revendication 4, caractérisé en ce que sa composition (H) en acides aminés est la suivante :

$$30$$
$$GLU \ ALA \ GLN \ THR \ GLU \ THR \ CYS \ THR \ VAL \ ALA \ PRO \ ARG$$
$$(H)$$

11. Peptide selon la revendication 5, caractérisé en ce que sa composition (J) en acides aminés est la suivante :

$$40 \qquad\qquad\qquad\qquad\qquad 50$$
$$GLN \ ASN \ CYS \ GLY \ PHE \ PRO \ GLY \ VAL \ THR \ PRO \ SER \ GLN \ CYS \ ALA \ ASN \ LYS$$
$$(J)$$

12. Peptide selon la revendication 6, caractérisé en ce que sa composition (K) en acides aminés est la suivante :

$$60$$
$$GLY \ CYS \ CYS \ PHE \ ASP \ ASP \ THR \ VAL \ ARG$$
$$(K)$$

13. Peptide selon la revendication 7, caractérisée en ce que sa composition (L) en acides aminés est la suivante :

$$70$$
$$GLY \ VAL \ PRO \ TRP \ CYS \ PHE \ TYR \ PRO \ ASN \ THR \ ILE \ ASP \ VAL \ PRO$$
$$80$$
$$PRO \ GLU \ GLU \ GLU \ CYS \ GLU \ PHE$$
$$(L)$$

14. Peptide selon la revendication 9, caractérisé en ce que sa composition (E) en amino acides est la sui-

vante :

```
                          30
GLU  ALA  GLN  THR  GLU  THR  CYS  THR  VAL  ALA  PRO  ARG  GLU  ARG
         40                                          50
GLN  ASN  CYS  GLY  PHE  PRO  GLY  VAL  THR  PRO  SER  GLN  CYS  ALA
ASN  LYS
                                    (E)
```

15. Peptide caractérisé en ce qu'il est constitué par un fragment correspondant aux 28 acides aminés de l'extrémité C-terminale du peptide (D) selon la revendication 2, en ce qu'il est d'un poids moléculaire d'environ 3100 Da, en ce que sa composition (F) en amino acides est la suivante :

```
     60                                                          70
CYS  PHE  ASP  ASP  THR  VAL  ARG  GLY  VAL  PRO  TRP  CYS  PHE  TYR
                                         80
PRO  ASN  THR  ILE  ASP  VAL  PRO  PRO  GLU  GLU  GLU  CYS  GLU  PHE,
                                    (F)
```

en ce qu'il correspond à une partie de la forme endogène sécrétée du peptide de séquence (D) et en ce qu'il permet la détection du peptide de formule (D) éventuellement présent dans un organe objet d'un dépistage ou d'un diagnostic.

16. Anticorps polyclonaux anti-pS2, caractérisés en ce qu'ils sont obtenus par immunisation de lapins ou autres animaux appropriés, contre la protéine pS2 ou ses fragments, selon l'une quelconque des revendications 1 à 15.

17. Anticorps monoclonaux anti-pS2, caractérisés en ce qu'ils sont obtenus par clonage d'hybridomes résultant de la fusion de cellules de rates de mammifères appropriés immunisés par des injections convenables de la protéine pS2 ou de fragments de celle-ci, selon l'une quelconque des revendications 1 à 15, avec des cellules d'un myélome approprié.

18. Procédé d'obtention du peptide de formule (D), caractérisé en ce qu'on précipite les protéines contenues dans un liquide biologique par de l'acétone à une basse température, on purifie le culot renfermant le peptide par chromatographie pour récupérer les fractions contenant le peptide, qui sont éventuellement congelées et/ou concentrées.

19. Procédé d'obtention du peptide de formule (D), selon la revendication 18, caractérisé en ce que préalablement à la purification du liquide d'origine biologique, on part de cellules MCF-7 qui sont mises en culture en présence d'oestradiol, le surnageant de culture est recueilli, puis soumis au processus de purification selon la revendication 18.

20. Procédé d'obtention du peptide de formule (D) selon la revendication 18, caractérisé en ce que le liquide biologique mis en oeuvre est du suc gastrique contenant ledit peptide sécrété par les tissus gastriques, qui est neutralisé par un tampon approprié de purification à pH 9,6 environ, avant d'être soumis au processus de purification selon la revendication 18.

21. Procédé selon l'une quelconque des revendications 18 à 20, caractérisé en ce que le peptide de formule (D) est isolé par immuno-purification à partir de liquides biologiques ou d'extraits de cellules, à l'aide d'anticorps dirigés contre la protéine pS2 ou contre des fragments de celle-ci selon l'une quelconque des revendications 1 à 15.

22. Procédé de préparation de la protéine pS2 et de fragments de celle-ci selon l'une quelconque des revendications 1 à 15, par voie de synthèse, notamment par synthèse chimique ou par biosynthèse.

23. Méthode de dépistage et de diagnostic de l'état pathologique d'un organe qui provoque l'expression par ledit organe, du peptide de formule (D) ou de ses précurseurs ou de ses fragments, caractérisée en ce que la présence d'un de ces peptides est déterminée par méthode immunologique dans des prélèvements biologiques, à l'aide d'anticorps selon l'une quelconque des revendications 16 et 17.

24. Méthode de dépistage et de diagnostic selon la revendication 23, caractérisée en ce que la présence du peptide (D) est détectée à l'aide d'anticorps dirigés contre le peptide (F).

25. Méthode suivant la revendication 23 ou la revendication 24, caractérisée en ce qu'on effectue le dépistage et le diagnostic sur des fluides corporels.

26. Méthode selon la revendication 23 ou la revendication 24, caractérisée en ce que l'expression du gène pS2 est déterminée par immunocytochimie.

27. Méthode selon la revendication 23 ou la revendication 24, caractérisée en ce que l'expression du gène pS2 est déterminée dans des prélèvements de tumeurs.

28. Méthode de détection et de diagnostic selon la revendication 27, caractérisée en ce que les prélèvements de tumeurs mis en oeuvre sont des coupes histologiques.

29. Méthode selon la revendication 28, caractérisée en ce que les prélèvements de tumeurs mis en oeuvre sont des coupes histologiques conservées et stabilisées par enrobage dans de la paraffine et fixées à la formaline.

30. Méthode selon la revendication 27, caractérisée en ce que les prélèvements de tumeurs mis en oeuvre sont des cytosols.

31. Méthode selon la revendication 26, caractérisée en ce que la méthode immunocytochimique est mise en oeuvre par incubation desdits prélèvements avec des anticorps de lapin, ou autre animal approprié, poly- ou monoclonaux anti-pS2 ou anti-fragments de pS2, puis avec un système de marquage et de révélation approprié de l'anticorps.

32. Méthode selon l'une quelconque des revendications 23 à 31 , caractérisée en ce que des prélèvements biologiques utilisés pour la détection d'une tumeur du sein sont soumis à une détection immunologique simultanée des RE, des RP et de la protéine pS2 ou d'un de ses fragments, faisant apparaître d'une part, une hétérogénéité fonctionnelle additionnelle dans les cancers du sein RE(+) (avec 60% environ seulement de tumeurs RE(+) également positives pour les RP et l'expression du gène pS2, et d'autre part, une grande homogénéité des cancers RE(-) en ce qui concerne les RP et l'expression du gène pS2 (avec environ 96% d'absence d'expression de ces deux gènes inductibles par des oestrogènes).

33. Procédé de dépistage et de diagnostic selon l'une quelconque des revendications 23 à 32, appliqué à la détection d'états pathologiques de l'estomac.

34. Méthode de dépistage et de diagnostic selon l'une quelconque des revendications 23 à 33, caractérisée en ce que l'expression du gène pS2 est déterminée par scintigraphie à l'aide d'au moins un anticorps selon l'une quelconque des revendications 16 et 17.

35. Kit prêt à l'emploi pour le dépistage et le diagnostic selon l'une quelconque des revendications 23 à 26 et 28 à 34 de l'état pathologique d'un organe, par dosage immunologique dans des prélèvements de tumeurs tels que coupes histologiques ou cytosols ou dans les fluides corporels tels que notamment les urines, caractérisé en ce qu'il comprend :
- des doses appropriées d'anticorps polyclonaux ou monoclonaux dirigés contre la protéine pS2 et/ou ses fragments ;
- des doses appropriées de protéine pS2 et/ou de ceux de ses fragments contre lesquels sont dirigés les anticorps mis en oeuvre ;
- des doses appropriées d'anticorps anti-immunoglobulines de lapin (ou d'un autre mammifère approprié) marqués de façon appropriée ;
- un système de révélation de la réaction anticorps-antigène constitué par un deuxième anticorps dirigé contre un épitope de la protéine pS2, marqué de façon appropriée ;
- éventuellement une substance de coloration des cellules tumorales, si le prélèvement biologique est constitué par des coupes histologiques et si la coloration de celles-ci est désirée ;
- des quantités utiles de tampons appropriés pour la mise en oeuvre du test de dépistage et de diagnostic.

36. Agents thérapeutiques, caractérisés en ce qu'ils contiennent un ou plusieurs anticorps selon l'une quelconque des revendications 17 et 18.

37. Agents thérapeutiques selon la revendication 36 caractérisés en ce qu'ils sont associés à un autre agent thérapeutique.

**Claims**

1. Peptide, characterised in that it is identified as being the secreted form of the protein pS2, in that it consists of a fragment corresponding to 60 amino acids of the said protein pS2, in that it starts with the sequence GLU - ALA - GLN, GLU being situated in position twenty five after the N-terminal MET of the protein pS2 and in that its molecular weight is about 6,600 Daltons, the said peptide being so-called "peptide pS2".

2. Peptide according to Claim 1, characterised in that its amino acid sequence (D) is the following:

```
                           30
GLU  ALA  GLN  THR  GLU  THR  CYS  THR  VAL  ALA  PRO  ARG  GLU  ARG
     40
GLN  ASN  CYS  GLY  PHE  PRO  GLY  VAL  THR  PRO  SER  GLN  CYS  ALA
                                            60
ASN  LYS  GLY  CYS  CYS  PHE  ASP  ASP  THR  VAL  ARG  GLY  VAL  PRO
               70                                          80
TRP  CYS  PHE  TYR  PRO  ASN  THR  ILE  ASP  VAL  PRO  PRO  GLU  GLU
GLU  CYS  GLU  PHE

                                        (D)
```

3. Peptide according to either of Claims 1 and 2, characterised in that it comprises four trypsin-cleaving sites which are situated after the basic amino acids $Arg_{36}$, $Arg_{38}$, $Lys_{54}$ and $Arg_{63}$.

4. Peptide characterised in that it corresponds to the 12 N-terminal amino acids of the peptide of sequence (D) according to Claim 2.

5. Peptide characterised in that it corresponds to the amino acid sequence ranging from $GLN_{39}$ to $LYS_{54}$, of the peptide of sequence (D) according to Claim 2.

6. Peptide characterised in that it corresponds to the amino acid sequence ranging from $GLY_{55}$ to $ARG_{63}$ of the peptide of sequence (D) according to Claim 2.

7. Peptide characterised in that it corresponds to the 21 C-terminal amino acids of the peptide of sequence (D) according to Claim 2.

8. Peptide characterised in that it consists of a fragment corresponding to the 31 C-terminal amino acids of the peptide pS2 according to Claim 2 and in that it has a molecular weight of about 3450 Da.

9. Peptide characterised in that it consists of a fragment corresponding to the 30 N-terminal amino acids of the peptide pS2 according to Claim 2 and in that it has a molecular weight of about 3300 Da.

**10.** Peptide according to Claim 4, characterised in that its amino acid composition ( H ) is the following:

$$
\begin{array}{l}
30 \\
\text{GLU ALA GLN THR GLU THR CYS THR VAL ALA PRO ARG} \\
\hspace{12cm} \text{( H )}
\end{array}
$$

**11.** Peptide according to Claim 5, characterised in that its amino acid composition (J) is the following:

$$
\begin{array}{l}
40 \hspace{7cm} 50 \\
\text{GLN ASN CYS GLY PHE PRO GLY VAL THR PRO SER GLN CYS ALA} \\
\text{ASN LYS} \\
\hspace{9cm} \text{( J )}
\end{array}
$$

**12.** Peptide according to Claim 6, characterised in that its amino acid composition (K) is the following:

$$
\begin{array}{l}
60 \\
\text{GLY CYS CYS PHE ASP ASP THR VAL ARG} \\
\hspace{9cm} \text{( K )}
\end{array}
$$

**13.** Peptide according to Claim 7, characterised in that its amino acid composition (L) is the following:

$$
\begin{array}{l}
70 \\
\text{GLY VAL PRO TRP CYS PHE TYR PRO ASN THR ILE ASP VAL PRO} \\
\hspace{2cm} 80 \\
\text{PRO GLU GLU GLU CYS GLU PHE} \\
\hspace{8cm} \text{( L )}
\end{array}
$$

**14.** Peptide according to Claim 9, characterised in that its amino acid composition (E) is the following:

$$
\begin{array}{l}
30 \\
\text{GLU ALA GLN THR GLU THR CYS THR VAL ALA PRO ARG GLU ARG} \\
\hspace{2cm} 40 \hspace{7cm} 50 \\
\text{GLN ASN CYS GLY PHE PRO GLY VAL THR PRO SER GLN CYS ALA} \\
\text{ASN LYS} \\
\hspace{8cm} \text{( E )}
\end{array}
$$

**15.** Peptide characterised in that it consists of a fragment corresponding to the 28 amino acids of the C-terminal end of the peptide (D) according to Claim 2, in that it has a molecular weight of about 3100 Da, in that its amino acid composition (F) is the following:

60                                                                70

CYS PHE ASP ASP THR VAL ARG GLY VAL PRO TRP CYS PHE TYR

80

PRO ASN THR ILE ASP VAL PRO PRO GLU GLU GLU CYS GLU PHE

(F)

and in that it corresponds to a portion of the secreted endogenous form of the peptide of sequence (D) and in that it allows the detection of the peptide of formula (D) which may be present in an organ which is the target of a screening or a diagnosis.

16. Anti-pS2 polyclonal antibodies characterised in that they are obtained by immunisation of rabbits or other appropriate animals against the protein pS2 or fragments thereof according to any one of Claims 1 to 15.

17. Anti-pS2 monoclonal antibodies characterised in that they are obtained by cloning hybridomas resulting from the fusion of spleen cells of appropriate mammals immunised by suitable injections of the protein pS2 or fragments thereof according to any one of Claims 1 to 15, with appropriate myeloma cells.

18. Process for producing the peptide of formula (D), characterised in that the proteins contained in a biological fluid are precipitated with acetone at low temperature, the pellet containing the peptide is purified by chromatography in order to recover the peptide-containing fractions which are optionally frozen and/or concentrated.

19. Process for producing the peptide of formula (D), according to Claim 18, characterised in that prior to the purification of the fluid of biological origin, MCF-7 cells, which are cultured in the presence of oestradiol, are used as starting material, the culture supernatant is recovered and then subjected to the purification process according to Claim 18.

20. Process for producing the peptide of formula (D) according to Claim 18, characterised in that the biological fluid used is gastric juice containing the said peptide secreted by the gastric tissues, which is neutralised with an appropriate purification buffer at about pH 9.6, before being subjected to the purification process according to Claim 18.

21. Process according to any one of Claims 18 to 20, characterised in that the peptide of formula (D) is isolated by immunopurification from biological fluids or cellular extracts, using antibodies directed against the protein pS2 or against fragments thereof according to any one of Claims 1 to 15.

22. Process for preparing the protein pS2 and fragments thereof according to any one of Claims 1 to 15, by the synthesis route, in particular by chemical synthesis or by biosynthesis.

23. Method for screening and diagnosing the pathological state of an organ which brings about the expression by the said organ of the peptide of formula (D) or of precursors or fragments thereof, characterised in that the presence of one of these peptides is determined in biological samples by an immunological method using antibodies according to either of Claims 16 and 17.

24. Screening and diagnostic method according to Claim 23, characterised in that the presence of the peptide (D) is detected using antibodies directed against the peptide (F).

25. Method according to Claim 23 or Claim 24, characterised in that the screening and diagnosis are performed on body fluids.

26. Method according to Claim 23 or Claim 24, characterised in that the expression of the pS2 gene is determined by immunocytochemistry.

27. Method according to Claim 23 or Claim 24, characterised in that the expression of the pS2 gene is determined on tumour samples.

28. Detection and diagnostic method according to Claim 27, characterised in that the tumour samples used are histological sections.

29. Method according to Claim 28, characterised in that the tumour samples used are histological sections preserved and stabilised by coating with paraffin and fixed using formalin.

30. Method according to Claim 27, characterised in that the tumour samples used are cytosols.

31. Method according to Claim 26, characterised in that the immunocytochemical method is carried out by incubating the said samples with anti-pS2 or anti-pS2 fragment poly-or monoclonal antibodies from rabbits or another appropriate animal, and then with an appropriate antibody labelling and detection system.

32. Method according to any one of Claims 23 to 31, characterised in that the biological samples used for detection of a breast tumour are subjected to a simultaneous immunological detection of the ERs, PRs and of the protein pS2 or fragments thereof, revealing on the one hand, an additional functional heterogeneity in ER(+) breast cancers (with only about 60% of the ER(+) tumours also positive for the PRs and the expression of the pS2 gene), and on the other hand, a high homogeneity of the ER(-) cancers with respect to the PRs and the expression of the pS2 gene (with about 96% absence of expression of these two oestrogen-inducible genes),

33. Screening and diagnostic process according to any one of Claims 23 to 32, applied to the detection of the pathological states of the stomach.

34. Screening and diagnostic method according to any one of Claims 23 to 33, characterised in that the expression of the pS2 gene is determined by scintigraphy using at least one antibody according to either of Claims 16 and 17.

35. Ready-for-use kit for screening and diagnosing according to any one of Claims 23 to 26 and 28 to 34 the pathological state of an organ, by immunological assay on tumour samples such as histological sections or cytosols or on body fluids such as in particular urines, characterised in that it comprises:
   - appropriate doses of polyclonal or monoclonal antibodies directed against the protein pS2 and/or fragments thereof;
   - appropriate doses of protein pS2 and/or of those of fragments thereof against which the antibodies used are directed;
   - appropriate doses of appropriately labelled anti-rabbit (or another appropriate mammal) immunoglobulin antibodies;
   - a system for detecting the antibody-antigen reaction consisting of a second appropriately labelled antibody directed against an epitope of the protein pS2;
   - optionally, a substance for staining tumour cells, if the biological sample consists of histological sections and if the staining of the latter is desired;
   - suitable amounts of buffers appropriate for carrying out the screening and diagnostic test.

36. Therapeutic agents, characterised in that they contain one or more antibodies according to either of Claims 17 and 18.

37. Therapeutic agents according to Claim 36, characterised in that they are combined with another therapeutic agent.

**Patentansprüche**

1. Peptid, dadurch **gekennzeichnet**, daß es als die Sekretionsform des Proteins pS2 identifiziert ist, es aus einem Fragment entsprechend 60 Aminosaüren des Proteins pS2 besteht, es mit der Sequenz GLU - ALA - GLN beginnt, wobei das GLU an der 25. Position nach dem N-terminalen MET des Proteins pS2 angeordnet ist, und daß sein Molekulargewicht etwa 6 600 Dalton beträgt, wobei das Peptid als "Peptid pS2" bezeichnet wird.

2. Peptid nach Anspruch 1, dadurch **gekennzeichnet,** daß es die folgende Aminosäuresequenz (D) besitzt:

```
                              30
GLU ALA GLN THR GLU THR CYS THR VAL ALA PRO ARG GLU ARG
                              40
GLN ASN CYS GLY PHE PRO GLY VAL THR PRO SER GLN CYS ALA
                                              60
ASN LYS GLY CYS CYS PHE ASP ASP THR VAL ARG GLY VAL PRO
                  70                                      80
TRP CYS PHE TYR PRO ASN THR ILE ASP VAL PRO PRO GLU GLU
GLU CYS GLU PHE
```

(D)

3. Peptid nach einem der Ansprüche 1 und 2, dadurch **gekennzeichnet,** daß es vier Trypsin-Spaltstellen besitzt, die nach den basischen Aminosäuren $Arg_{36}$, $Arg_{38}$, $Lys_{54}$ und $Arg_{63}$ angeordnet sind.

4. Peptid, dadurch **gekennzeichnet**, daß es 12 N-terminalen Aminosäuren des Peptids der Sequenz (D) nach Anspruch 2 entspricht.

5. Peptid, dadurch **gekennzeichnet,** daß es der Aminosäuresequenz von $GLN_{39}$ bis $LYS_{54}$ des Peptids der Sequenz (D) nach Anspruch 2 entspricht.

6. Peptid, dadurch **gekennzeichnet,** daß es der Aminosäuresequenz von $GLY_{55}$ bis $ARG_{63}$ des Peptids der Sequenz (D) nach Anspruch 2 entspricht.

7. Peptid, dadurch **gekennzeichnet,** daß es die 21 C-terminalen Aminosäuren des Peptids der Sequenz (D) nach Anspruch 2 entspricht.

8. Peptid, dadurch **gekennzeichnet,** daß es aus einem Fragment entsprechend den 31 C-terminalen Aminosäuren des Peptids pS2 nach Anspruch 2 besteht und daß es ein Molekulargewicht von etwa 3 450 Da besitzt.

9. Peptid, dadurch **gekennzeichnet,** daß es aus einem Fragment entsprechend den 30 N-terminalen Aminosäuren des Peptids pS2 nach Anspruch 2 besteht und daß es ein Molekulargewicht von etwa 3 300 Da besitzt.

10. Peptid nach Anspruch 4, dadurch **gekennzeichnet,** daß es die folgende Aminosäurezusammensetzung (H) besitzt:

```
                      30
GLU ALA GLN THR GLU THR CYS THR VAL ALA PRO ARG
```

(H)

11. Peptid nach Anspruch 5, dadurch **gekennzeichnet,** daß es die folgende Aminosäurezusammensetzung (J) besitzt:

```
        40                                      50
GLN ASN CYS GLY PHE PRO GLY VAL THR PRO SER GLN CYS ALA ASN LYS
```

(J)

**12.** Peptid nach Anspruch 6, dadurch **gekennzeichnet,** daß es die folgende Aminosäurezusammensetzung (K) besitzt:

$$60$$
$$\text{GLY CYS CYS PHE ASP ASP THR VAL ARG}$$
$$(K)$$

**13.** Peptid nach Anspruch 7, dadurch **gekennzeichnet,** daß es die folgende Aminosäurezusammensetzung (L) besitzt:

$$70$$
$$\text{GLY VAL PRO TRP CYS PHE TYR PRO ASN THR ILE ASP VAL PRO}$$
$$80$$
$$\text{PRO GLU GLU GLU CYS GLU PHE}$$
$$(L)$$

**14.** Peptid nach Anspruch 9, dadurch **gekennzeichnet,** daß es die folgende Aminosäurezusammensetzung (E) besitzt:

$$30$$
$$\text{GLU ALA GLN THR GLU THR CYS THR VAL ALA PRO ARG GLU ARG}$$
$$40 \qquad\qquad\qquad\qquad 50$$
$$\text{GLN ASN CYS GLY PHE PRO GLY VAL THR PRO SER GLN CYS ALA}$$
$$\text{ASN LYS}$$
$$(E)$$

**15.** Peptid, dadurch **gekennzeichnet,** daß es aus einem Fragment entsprechend den 28 Aminosäuren des C-terminalen Endes von Peptid (D) nach Anspruch 2 besteht, es ein Molekulargewicht von etwa 3 100 Da besitzt, es die folgende Aminosäurezusammensetzung (F) besitzt:

$$60 \qquad\qquad\qquad\qquad\qquad\qquad\qquad 70$$
$$\text{CYS PHE ASP ASP THR VAL ARG GLY VAL PRO TRP CYS PHE TYR}$$
$$80$$
$$\text{PRO ASN THR ILE ASP VAL PRO PRO GLU GLU GLU CYS GLU PHE,}$$
$$(F)$$

es einem Teil der endogenen sekretierten Form des Peptids der Sequenz (D) entspricht und daß es die Detektion des Peptids der Formel (D), das gegebenenfalls in einem Organ, das eines Auspüren oder einer Diagnose unterworfen ist, vorhanden ist, gestattet.

**16.** Anti-pS2-Polyclonale-Antikörper, dadurch **gekennzeichnet,** daß sie durch Immunisieren von Kaninchen oder anderen geeigneten Tieren,gegen das Protein pS2 oder seine Fragmente nach einem der Ansprüche 1 bis 15 erhalten worden sind.

**17.** Anti-pS2-Monoclonale-Antikörper, dadurch **gekennzeichnet,** daß sie durch Clonieren von Hybridomen die aus der Fusion von Milzzellen von geeigneten Säugetieren, die mit geeigneten Injektionen des Proteins pS2 oder dessen Fragmenten nach einem der Ansprüche 1 bis 15 immunisiert worden sind, mit geeigneten Myelomazellen, erhalten worden sind.

18. Verfahren zur Gewinnung des Peptids der Formel (D), dadurch **gekennzeichnet,** daß man die man einer biologischen Flüssigkeit enthaltenen Proteine mit Aceton bei einer tiefen Temperatur ausfällt, den das Peptid enthaltenden Niederschlag chromatographisch reinigt, um die peptidhaltigen Fraktionen zu gewinnen, welche gegebenenfalls eingefroren und/oder konzentriert werden.

19. Verfahren zur Gewinnung des Peptids der Formel (D), nach Anspruch 18, dadurch **gekennzeichnet,** daß man vor der Reinigung der biologischen Ausgangsflüssigkeit von MCF-7-Zellen ausgeht, die in Gegenwart von Östradiol gezüchtet werden, der Kulturüberstand gewonnen wird, anschließend einem Reinigungsverfahren nach Anspruch 18 unterworfen wird.

20. Verfahren zur Gewinnung des Peptids der Formel (D) nach Anspruch 18, dadurch **gekennzeichnet,** daß die verwendete biologische Flüssigkeit Magensaft ist, der das von den Magengeweben sekretierte Peptid enthält, der durch einen geeigneten Reinigungspuffer bei einem pH-Wert von etwa 9,6 neutralisiert wird vor dem Reiningungsverfahren nach Anspruch 18.

21. Verfahren nach einem der Ansprüche 18 bis 20, dadurch **gekennzeichnet,** daß das Peptid der Formel (D) durch Immun-Reinigung aus biologischen Flüssigkeiten oder Zellextrakten mit Hilfe von Antikörpern gegen das Protein pS2 oder gegen dessen Fragmente nach einem der Ansprüche 1 bis 15, isoliert wird.

22. Verfahren zur Herstellung des Proteins pS2 und dessen Fragmente nach einem der Ansprüche 1 bis 15 mittels Synthese, insbesondere mittels chemischer Synthese oder Biosynthese.

23. Verfahren zum Auspüren und zur Diagnose des pathologischen Zustands eines Organs, der die Expression des Peptids der Formel (D) oder seiner Vorläufer oder seiner Fragmente durch dieses Organ hervorruft, dadurch **gekennzeichnet,** daß das Vorliegen eines dieser Peptide mittels eines immunologischen Verfahrens in entnommenen biologischen Proben mit Hilfe der Antikörper nach einem der Ansprüche 16 und 17 bestimmt wird.

24. Verfahren zum Auspüren und zur Diagnose nach Anspruch 23, dadurch **gekennzeichnet,** daß das Vorliegen des Peptids (D) mit Hilfe von Antikörpern gegen das Peptid (F) detektiert wird.

25. Verfahren nach Anspruch 23 oder 24, dadurch **gekennzeichnet,** daß man das Auspüren und die Diagnose an Körperflüssigkeiten vornimmt.

26. Verfahren nach Anspruch 23 oder 24, dadurch **gekennzeichnet,** daß die Expression des pS2-Gens immuncytochemisch bestimmt wird.

27. Verfahren nach Anspruch 23 oder 24, dadurch **gekennzeichnet,** daß die Expression des pS2-Gens in Abstrichen von Tumoren bestimmt wird.

28. Verfahren zur Detektion und zur Diagnose nach Anspruch 27, dadurch **gekennzeichnet,** daß die verwendeten Abstriche von Tumoren histologische Schnitte sind.

29. Verfahren nach Anspruch 28, dadurch **gekennzeichnet,** daß die verwendeten Abstriche von Tumoren durch Einbettung in Paraffin konservierte und stabilisierte und in Formalin fixierte histologische Schnitte sind.

30. Verfahren nach Anspruch 27, dadurch **gekennzeichnet,** daß die verwendeten Abstriche von Tumoren Cytosole sind.

31. Verfahren nach Anspruch 26, dadurch **gekennzeichnet,** daß das immuncytochemische Verfahren durch Inkubation diesen Abstriche mit Anti-pS2-poly-oder monoclonalen-Antikörpern oder Anti-fragmenten von pS2, eines Kaninchens oder eines anderen geeigneten Tieres und anschließend mit einem geeigneten Markierungs- and Detektionssystem den Antikörper durchgeführt wird.

32. Verfahren nach einem der Ansprüche 23 bis 31, dadurch **gekennzeichnet,** daß die zur Detektion eines Brustkrebses verwendeten biologischen Abstrichen einer gleichzeitigen immunologischen Detektion auf RE, auf RP und das Protein pS2 oder eines seiner Fragmente unterworfen werden, was einerseits eine zusätzliche funktionelle Heterogenität in den Brustkrebsen RE(+) (wobei nur etwa 60% der RE(+)-Tumore ebenso auf RP als auch auf die Expression des pS2-Gens positiv sind) und andererseits eine große Homogenität der RE(-)-Krebsarten betreffend die RP und die Expression des pS2-Gens (mit etwa 96% Abwesenheit der Expression dieser beiden durch Östrogene induzierbaren Gene), zeigt.

33. Verfahren zum Auspüren und zur Diagnose nach einem der Ansprüche 23 bis 32, dadurch **gekennzeichnet,** daß es auf die Detektion pathologischer Zustände des Magens angewandt wird.

34. Verfahren zum Auspüren und Diagnose nach einem der Ansprüche 23 bis 33, dadurch **gekennzeichnet,** daß die Expression des pS2-Gens szintigraphisch mit Hilfe mindestens eines Antikörpers nach einem der Ansprüche 16 und 17 bestimmt wird.

35. Kit, bereit zur Verwendung bei dem Auspüren und der Diagnose nach einem der Ansprüche 23 bis 26 und 28 bis 34 eines pathologischen Zustands eines Organs durch immunologische Bestimmung in Tumor Abstrichen , wie histologischen Schnitten oder Cytosolen, oder in Körperflüssigkeiten, wie insbesondere Urin, dadurch **gekennzeichnet,** daß es
    - geeignete Mengen an polyclonalen oder monoclonalen Antikörpern gegen das Protein pS2 und/oder seine Fragmente;
    - geeignete Mengen des Proteins pS2 und/oder diejenigen seiner Fragmente gegen die die verwendeten Antikörper gerichtet sind;
    - geeignete Mengen an Anti-Immunglobulin-Antikörpern von Kaninchen (oder einem anderen geeigneten Säugetier), die geeignet markiert sind;
    - ein System zur Entwicklung der Antikörper-Antigen-Reaktion, das aus einem zweiten Antikörper, der gegen ein Epitop des Proteins pS2 gerichtet ist und geeigneterweise markiert ist, besteht;
    - gegebenenfalls eine Substanz zum Anfärben der Tumorzellen, wenn das biologische Abstrich aus histologischen Schnitten besteht und wenn deren Anfärbung gewünscht ist;
    - nützliche Mengen an geeigneten Puffern zur Durchführung des Tests zur Untersuchung und zur Diagnose umfaßt.

36. Therapeutische Mittel, dadurch **gekennzeichnet,** daß sie einen oder mehrere Antikörper nach einem der Ansprüche 17 und 18 umfassen.

37. Therapeutische Mittel nach Anspruch 36, dadurch **gekennzeichnet,** daß sie zusammen mit einem anderen therapeutischen Mittel vorliegen.

# FIG. 1

$^{35}$S – CYSTEINE

A
MILIEU

B
EXTRAITCELLULAIRE

C
PEPTIDE
SYNTHETIQUE (C)

FIG.2

# FIG.3

## FIG.4

FIG.5

EP 0 345 315 B1

# FIG.6

```
1                                    10
MET ALA THR MET GLU ASN LYS VAL ILE CYS ALA LEU VAL LEU VAL SER MET LEU ALA
                                                                    ▼      ▼
20                  24                  30
LEU GLY THR LEU ALA GLU ALA GLN THR GLU THR CYS THR VAL ALA PRO ARG GLU ARG
                         1                                      12  13  14
            PS2_M
                    T1
            PS2_G

40                                  50              ▼
GLN ASN CYS GLY PHE PRO GLY VAL THR PRO SER GLN CYS ALA ASN LYS GLY CYS CYS
15                                                      30  31
                                                              T3
T4
          60            ▼                  ▽              70        ▽
PHE ASP ASP THR VAL ARG GLY VAL PRO TRP CYS PHE TYR PRO ASN THR ILE ASP VAL
              38  39  40              43  44              48  49
                                                 T2
                          T6                              T5
          80        84
PRO PRO GLU GLU GLU CYS GLU PHE
                        60
```